# EUROPEAN PATENT SPECIFICATION

(11) **EP 4 100 128 B1**
(45) Date of publication and mention of the grant of the patent: **09.04.2025**
(21) Application number: 21711056.8
(22) Date of filing: 08.02.2021
(51) Int. Cl.: A62B 7/10, A62B 9/00, A62B 18/00, A62B 23/02

(54) **DEVICE, SYSTEM AND METHOD FOR TREATING AIR DURING BREATHING**
VORRICHTUNG, SYSTEM UND VERFAHREN ZUR BEHANDLUNG VON LUFT WÄHREND DER ATMUNG
DISPOSITIF, SYSTÈME ET PROCÉDÉ DE TRAITEMENT DE L'AIR PENDANT LA RESPIRATION

(30) Priority: 09.02.2020 IL 27254920; 22.03.2020 IL 27354720; 05.07.2020 IL 27586320
(43) Date of publication of application: 14.12.2022
(73) Proprietor: PUREORR LTD., Savyon 5653827 (IL)
(72) Inventor: ATZMON, Yoav, 5653827 Savyon (IL); KERETH, Yefim, 7656836 Rehovot (IL)
(74) Representative: Gislon, Gabriele
(86) International application number: PCT/IL2021/050150
(87) International publication number: WO 2021/156876

(56) References cited:
- WO-A1-2016/112434
- WO-A1-2019/071296
- CN-A- 105 833 441
- CN-A- 106 693 226

## Description

### FIELD OF THE INVENTION

The present invention pertains generally to a device, system and method for treatment of inhaled and exhaled air and for monitoring air quality, system functionality and user health indicators. The inhaled and exhaled air treatment may be done either by filtering or by disinfection, or by gas absorption, or by any combination thereof.

### BACKGROUND OF THE INVENTION

Most air masks, currently in use, employ mechanical filters. These filters have a limited area, provide the user with limited protection, create a significant pressure drop that induces a peripheral leakage of contaminated or infected air, accumulate moisture and bad smell, put strain on the user's lungs causing the user to breathe more heavily, thus shortening the time period the user can suffer the inconvenience of such masks.

It is well-known that air in infected and toxic environments can be treated by either UV radiation, by filtering or by gas absorption. To employ UV radiation in a portable breathing device, extremely high energy efficiency is essential in order to conserve battery power. High energy efficiency implies low power consumption together with high efficacy. To employ a high level of filtering, an increased filter area is required, but it becomes a great challenge as portable breathing devices have only limited surface area. Effective gas absorption requires sufficient contact time, but this can be achieved only if the treated air flow matches the available space in the portable breathing device.

Some of these problems can be resolved by means of a blower that creates an active air flow, which reduces the pressure drop and makes the user's breathing easier. However, active air flow alone does not create a natural cycle of breathing since the volume of air supplied by the blower is not equal to the volume of the inhaled air, and likewise the volume of air drawn by the blower is not equal to the volume of the exhaled air. Active flow alone allows neither UV disinfection efficiency nor gas absorption contact time.

There is therefore a need to provide an inhalation and exhalation air treatment system to overcome the above drawbacks, to provide a high level of protection and to allow monitoring of the air quality, system functionality and the user's health indicators.

WO 2015/167098 discloses a mask having a body, an air purifier coupled to or formed on the body and configured to purify and discharge air introduced therein, and a control unit coupled to or formed on the body, and configured to control an air volume of the air purified and discharged through the air purifier.

WO 2017/192497 discloses a modular, portable, air purifier device, which may optionally include a UV filter capable of supplying filtered or otherwise conditioned airflow to an individual. It provides a good discussion of known techniques including negative pressure respirators, which typically take the form of either a mask, or a half mask respirator. The mask covers the nose and mouth and air is drawn through a filter by the negative pressure of inhalation. It is suggested that these types of masks increase respiratory stress because the user must overcome the air restriction presented by the air filter. It is further stated that a tight fit is essential to prevent unfiltered air from entering around the mask instead of through the filter. These types of masks also interfere with normal conversation because they cover both the nose and mouth.

WO/2008/070989 discloses a mask interface device for a protective mask of the type having a mask filter and a mask expiratory port, the mask expiratory port having an expiratory port valve of the type that is normally closed and openable upon expiration. An expiratory port interface assembly mountable to the mask expiratory port comprises at least one opening for venting expired gas to atmosphere. A one-way valve is positioned to control the flow of expired gas out through the opening, and is set to an opening pressure that provides positive end expiratory pressure.

US20100224193 discloses a breathing apparatus comprising a face mask that is coupled to an optional prefilter assembly that includes a housing containing a pre-filter filtration medium.

It is known that the efficiency of conventional face masks is limited owing, in no small part, to their low surface area, which is dictated by the fact that are worn on the face and must be reasonably comfortable. It is therefore known to enhance the filtering efficiency of face masks by coupling them to an external filter. Thus, it is known to adapt face masks for coupling to external elongate filters that are either integrated with the mask assembly or are in the form of cartridges that are worn on the back of the user and are coupled to the mask assembly via a flexible tubing.

Additionally, although it is known to use a one-way valve to control the flow of expired gas that is exhaled, it does not appear to be known to control the flow of air prior to inhalation.

CN 105 833 441 discloses a neck-hanging type air purification system comprising a gauze mask and a purification device, wherein the gauze mask covers the face of a human body, the purification device is used for purifying air, and besides, the gauze mask communicates with the purification device through an air inlet pipe.

CN 106 693 226 discloses a mask comprising a mask body and a first one-way air valve arranged on the mask body. Ventilation is conducted on the outer side of the mask through the first one-way air valve and is stopped on the inner side. A second one-way air valve is arranged on the mask body, and ventilation is conducted on the inner side of the mask through the second one-way air valve and is stopped on the outer side.

WO 2019/071296 discloses breathing apparatus having an elongate filter unit, one or more one-way valves, a mask assembly for covering the oral and nasal passage of a user and an electric blower for delivering a required air pressure and flow to the mask assembly. Air pressure is adjusted by controlling the blower using a breath-responsive flow control algorithm to control the air flow to the mask assembly. The blower can be a separate unit located behind the user's neck and fluidly coupled to the mask assembly by a pipe having a section of bellows at each end which is compressible and extensible to maintain a proper seal regardless of movement of the user's head.

### SUMMARY OF THE INVENTION

It is therefore one object of the present invention to provide a breathing apparatus that enhances the filtering efficiency of a regular face mask by means of a lightweight external filter cartridge that is coupled to the mask.

It is a further object of the present invention to provide a device and system for air treatment during inhalation or exhalation and for monitoring the air quality, the system functionality and the user health indicators.

These objects are realized in accordance with the invention by a device, system and method for treating the air during inhalation and exhalation having the features of the respective independent claims.

Thus, in accordance with one aspect of the invention there is provided a device for enhancing efficiency of an air mask wearable by a user to cover the mouth and nose of the user, the device comprising:
an elongated chamber fluidly couplable at a proximal end thereof to an air opening of the mask,
a filter element formed of flexible material that is wrapped around a sidewall of the chamber either internally or externally for filtering air flowing through the chamber in either direction,
a one-way valve mounted in association with the opening,
an air blower configured to continuously supply air for inhalation or to discharge exhaled air,
**characterized in that:**
   the device further comprises an expandable member fluidly coupled to the chamber, the one-way valve is configured for allowing treated air inhaled by the user to pass from the chamber to the mask or for allowing air exhaled by the user to pass from the mask to the chamber,
   the expandable member is operably coupled to the one-way valve for directing air through the filter during inhalation or exhalation;
   whereby if the opening is configured to be an air inlet, then during inhalation air is constrained to flow into the chamber and inflate the expandable member with treated air ready for inhalation prior to flowing out through the opening into the user's lungs; and
   if the opening is an air outlet, then during exhalation air is constrained to flow from the user's lungs into the chamber and inflate the expandable member with exhaled air prior to being discharged as treated air into the environment.

The device may be retrofitted to a mask to form an integral unit or the chamber may be adapted for attaching to an existing mask, typically but not necessarily via a screw-coupling. It can also be attached using a bayonet coupling or a push-fit snap connection or may be secured using clasps.

A system according to the invention includes the device with or without an integral mask in combination with auxiliary components as described in detail below.

### BRIEF DESCRIPTION OF THE DRAWINGS

In order to better understand the invention and its implementation in practice, embodiments will now be described, by way of non-limiting example only, with reference to the accompanying drawings, wherein
**Figs. 1A and 1B** illustrate basic embodiment of a UV disinfection system;
**Figs. 2A and 2B** illustrate a basic embodiment of the system of FIG. 1 at inhalation and exhalation stage;
**Figs. 3A to 3D** illustrate a basic embodiment of the system of FIG. 2 with an expandable member at a complete breathing cycle;
**Figs. 4A and 4B** illustrate a basic embodiment of the system of FIG. 3 with an extension pipe;
**Figs. 5A to 5C** illustrates an embodiment of the system of FIG. 4, with increased surface area filter;
**Fig. 6** **is** a more detailed cross-sectional view of an embodiment of the system of FIG. 5;
**Figs. 7A and 7B** illustrate an embodiment of the system of FIG. 5, with a different expandable member arrangement;
**Figs. 8A and 8B** illustrate an embodiment of the system of FIG. 7, with a head-wearable arrangement;
**Figs. 9A and 9B** illustrate an embodiment of the system of FIG. 7, with a soft neck - wearable, arrangement;
**Figs. 10A and 10B** illustrate an embodiment of the system of FIG. 7, with a rigid neck - wearable, arrangement;
**Figs. 11A and 11B** are cross-sectional views of the system of FIG. 10;
**Fig. 12** **is** a cross-sectional view of a power and monitoring module integrated with the system of FIG. 10;
**Figs. 13A and 13B** show pictorially the module of FIG. 12;
**Fig. 14** illustrates an embodiment of the system of FIG. 8, with a headband support;
**Fig. 15** illustrates an embodiment of the system of FIG. 8 and 14 with a neck support;
**Fig. 16** is a cross-section view of the system of FIG. 15;
**Figs. 17A and 17B** are perspective and cross-section views of electronic module and charcoal filter attached thereof, of the system of FIG. 15 and 16;
**Figs. 18A and 18B** illustrate left-side and right-side views of an embodiment of the system of FIG. 8 and 14; and
**Fig. 19** is a rear view of a mask for use with the system of FIG. 8, 14 and 18.

### DETAILED DESCRIPTION

In the following description of some embodiments, identical components that appear in more than one figure or that share similar functionality will be referenced by identical reference symbols.

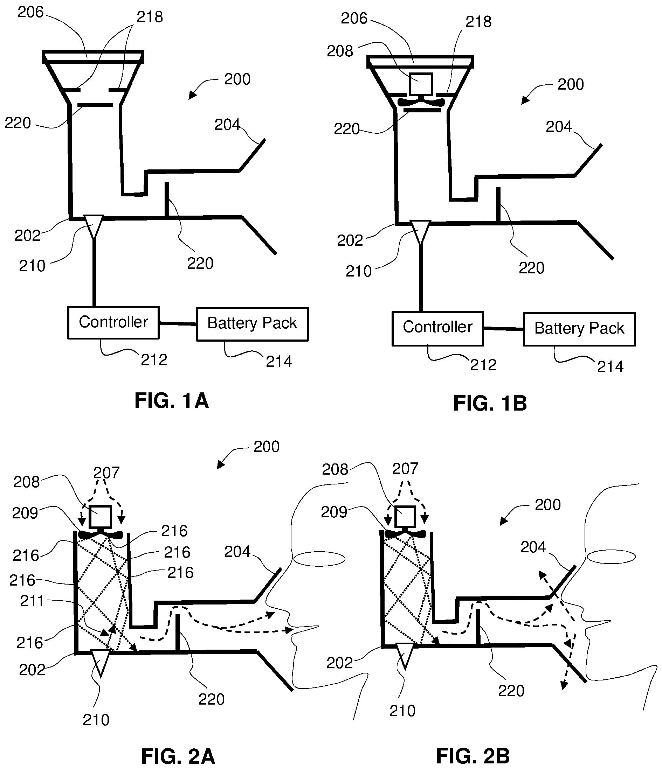

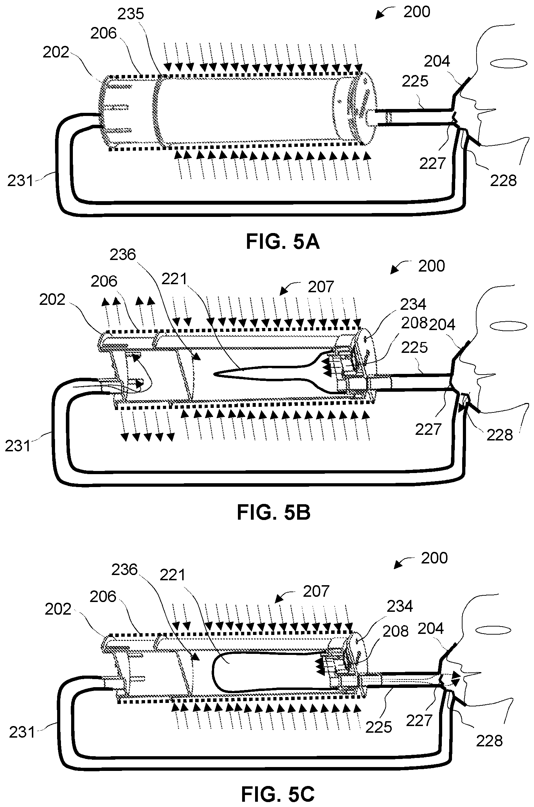

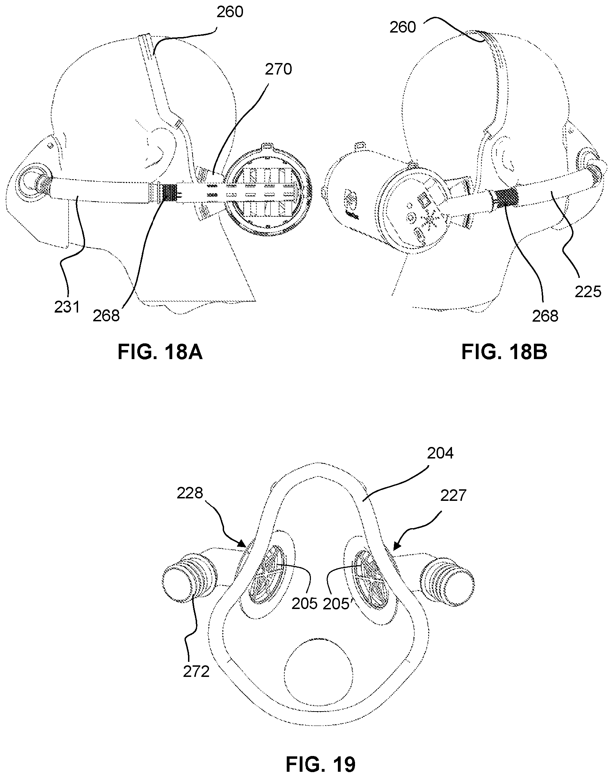

FIG. 1A illustrates a system 200 according to a first embodiment of the invention comprising a chamber 202 at least indirectly connected to a mask 204 having air openings 205 and 205" shown in Fig. 19, which serve as an air inlet and an air outlet, respectively. A filter element 206 configured to remove viruses and bacteria from either inhaled or exhaled air is mounted in association with the chamber 202. In the embodiment shown in the figures, the chamber is a right-circular cylinder and the filter element is likewise cylindrical and forms a hollow structure that surrounds an outer wall surface of the chamber. However, the geometry and location of the filter are not crucial so long as the filter is able to filter the air prior to its entering the chamber. So it could equally well be located inside the chamber in the form of a hollow tube around the internal wall of the chamber. The filter element 206 has a structure of a typical air filter, namely a particulate air filter, and possibly activated charcoal filter and another particulate filter to trap charcoal dust. The filter element 206 may also be formed from other filtering materials adapted to remove viruses and bacteria riding on particles. In this embodiment the filter element 206 is two dimensional and attached to the portion of the chamber 202 structure. In other embodiments (FIG. 5 to 8 and FIG. 14 to 16) the filter element 206 surrounds the chamber 202 to achieve a significantly larger filter surface area, thus reducing the velocity of air 207 (FIG. 2) over the filter material and thereby significantly increasing the filter efficiency.

The system 200 comprising the chamber, filter and an expandable member 221 described below may be a standalone device adapted for coupling to the mask 204. Alternatively, the device may be coupled to the mask to form an integrated system as shown in the figures and to which additional components may be coupled. For the sake of consistency, in the following description we will refer to the arrangement as a system regardless of whether or not it includes the mask as an integral component. FIG. 1B illustrates the system of FIG. 1A with an air blower 208, having blades 209 directed toward the interior of the chamber 202.

Both FIGS. 1A and 1B illustrate the system 200 with a UV source 210 exposed to the interior of the chamber 202. The UV source 210 is attached to one of the walls of the chamber 202 and is driven by a controller 212 powered by a battery pack 214. The controller 212 and the battery pack 214 may be integral with the system 200 or may be separate units that are carried by the user and mechanically and electrically coupled to the system via a suitable connector. The controller 212 can either switch the blower 208 and / or UV source 210 ON or OFF or adjust flow rate and power.

In this embodiment, the internal walls of the chamber 202 may be covered by a UV reflection layer, to allow multiple reflections 216 of the UV source rays 211. To retain the UV radiation energy inside the chamber, a baffle 218 and area obstacles 220 are located at the inlet and the outlet of the system 200 and cooperate to form a labyrinth that serves to reflect UV radiation internally between the inner walls of the chamber. The surfaces of the labyrinth 218 and 220 directed to the UV source 208 are covered by a UV reflecting layer, while the opposite surfaces of the labyrinth are covered by a UV absorption layer.

The surfaces of the blades 209 directed to the UV source 210 may also be covered by a UV reflection layer, allowing the blower 208 to reflect most of the direct rays 211 and reflected rays 216 hitting the blower blades 209 (FIG. 2).

FIG. 2A illustrates the system of FIG. 1B during inhalation. The air blower 208 is configured to provide a steady and continuous airflow 207 that meets the maximum breathing demand, thereby avoiding external air intake via the air mask 204. The power of the UV source 210 should, therefore, be much higher as the air volume to be disinfected is much larger than the actual air volume entering the lungs. The blower 208 creates an overpressure inside the mask, which dispels the entry of ambient air and thus obviates the need for a tight seal between the mask and the user's face.

FIG. 2B illustrates the system of FIG. 1B during exhalation, it being seen that the inlet air blower 208 maintains the same rate of airflow as in a previous inhalation. This configuration is therefore is not energy-efficient because the air blower 208 draws air into the system when the user inhales, when operation of the air blower is essential, and also when the user exhales, even though during exhalation there is no need for the blower 208 to draw air into the system.

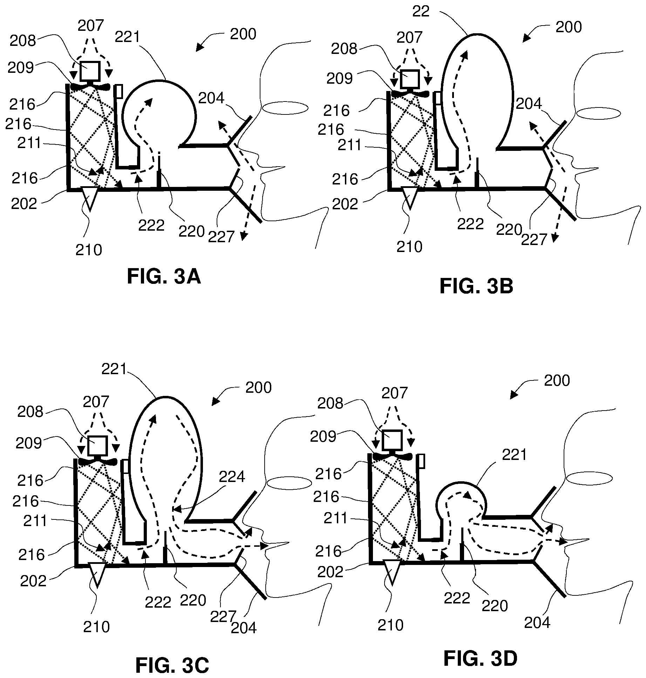

To overcome this disadvantage the embodiment of FIGS. 3A to 3D integrates an expandable member 221 into the system of FIGS. 1 and 2. The airflow 222, previously disinfected by UV source rays 211 in the chamber 202 inflates the expandable member 221 with a sufficient quantity of air ready for inhalation. Whenever the expandable member 221 achieves its maximum volume, it engages a sensor 223 that is interfaced to the system controller 212 and produces a switching signal. The controller 212 is responsive to the switching signal for switching OFF the air blower 208 and the UV source 210, thus saving the energy required from the battery pack 214. In this embodiment the inlet air blower 208 is configured to provide a relatively low and steady rate of airflow (about 25% only), as it should be lower than the maximum breathing flowrate (400 cc per sec), and therefore the power of the UV source 210 may be dramatically reduced. To provide the maximum breathing rate, the expandable member 221, previously inflated by the air blower 208, contains a sufficient quantity of already disinfected air and is ready to be deflated, by human inhalation.

FIG. 3A illustrates the system 200 with an expandable member 221 at an early stage of exhalation, when the expandable member 221 reaches its minimum volume after being deflated by human inhalation. In this embodiment the exhaled air is filtered by the filter layers of the air mask 204, the filter layers being structured to optimize the exhaled air filter.

FIG. 3B illustrates the system 200 with an expandable member 221 at a final stage of exhalation, when the expandable member 221 has reached its maximum volume.

FIG. 3C illustrates the system 200 with an expandable member 221 at an initial stage of inhalation, when the expandable member 221 is at its maximum volume.

FIG. 3D illustrates the system 200 at a final stage of inhalation, when the expandable member 221 is deflated and reaches its minimum volume.

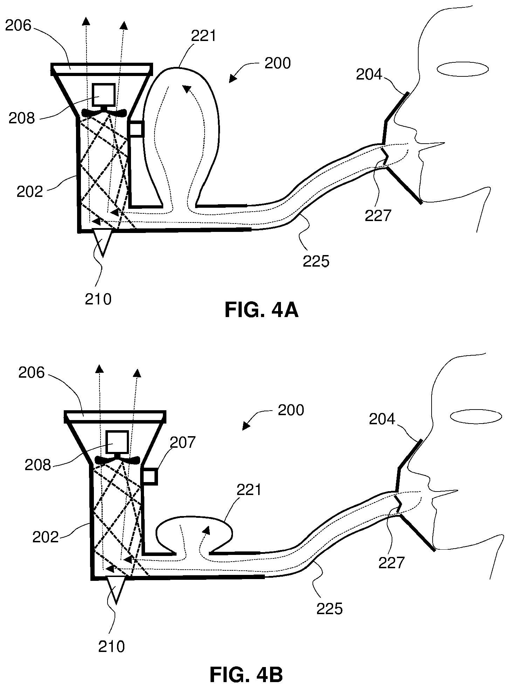

FIGS. 4A and 4B illustrate the system 200 of FIG. 3 having an expandable pipe 225 configured for exhalation air UV disinfection. In this configuration the air blower 208 is configured for an inverse airflow.

FIG. 4A shows the expandable member 221 at an early stage of inhalation, when it is fully inflated by exhalation of the previous breath, the air blower 210 moving possibly contaminated exhaled air via the treatment space of the chamber 202 under the UV radiation of the UV source 210.

FIG. 4B shows the expandable member 221 at the final stage of inhalation, when the expandable member 221 is fully deflated.

The embodiment of FIGS. 3 and 4 achieves high energy efficiency because the lower the air flow rate of the blower 208 through the chamber 202, the lower is the power of the UV source 210 required to disinfect the air as will now be explained.

Thus, typical inhalation-exhalation cycle time is about 5 seconds. Without the expandable member 221 the airflow rate would be determined by the nominal inhalation airflow rate, which is around 400 cc per sec. With the expandable member 221, there is about twice as much time to inflate the expandable member and therefore the airflow rate through the chamber 202 may be reduced by about 50%. If the airflow rate is reduced to half, the UV source 210 power may also be reduced by the same ratio.

It should be noted that the system configuration introduced in FIGS. 1 to 3 is optimal for uninfected people, as inhalation air quality provided by both the filtering element 206 and disinfection light of UV source 210 will be beyond the exhalation air quality provided by the air mask 204 filtering layers.

For infected people the system configuration of FIG. 4 may be more suitable, since the quality of the exhaled air provided by both the filtering element 206 and disinfection light of the UV source 210, will be higher than the quality of the inhaled air provided by the filtering layers of the air mask 204.

To allow the system 200 to be configurable, both for infected and uninfected people, a configurable one-way valve 227 shown in FIG. 3 and 4 may be provided. The flow-direction of the valve 227 can be easily changed by a user based on whether or not the user is infected. The air flow direction of the air blower 208 will be changed by the controller 212 accordingly to allow either the configuration of system 200 in FIGS. 1 to 3, or the configuration of system 200 in FIG. 4.

It will be appreciated that the expandable member 221 is not limited to the shapes, sizes and materials shown and described with reference to the drawings.

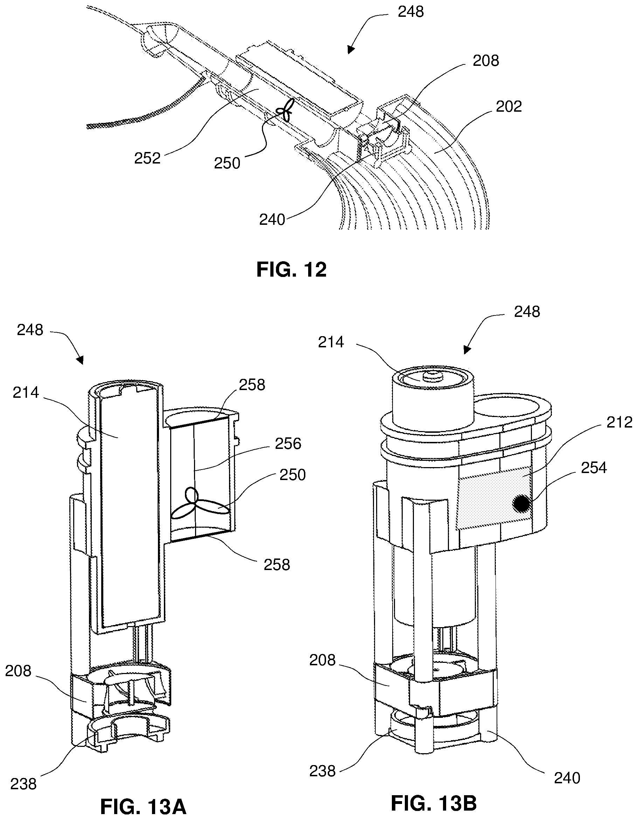

Operation of the system illustrated by FIGS. 1 to 4 may be monitored as follows: a) measure the airflow rate using an anemometer 250 (FIG. 12 and 13A); b) adjust the UV source 210 power based on the direction and flow rate of the breathing: switch the UV source 210 OFF at exhalation or adjust the UV source 210 power upon inhalation.

The power of the UV source 210 may be continuously monitored by a UV sensor (not shown) interfaced to the controller 212. The controller 212 may be configured to report system variables (e.g. UV source status, battery status) to a smartphone, computer or router via Bluetooth or WiFi communication to allow preventive maintenance and to allow advanced HMI (human-machine interface) with the system 200.

FIGS. 5A to 5C illustrate a system 200 according to a second embodiment having a peripheral filter 206. The peripheral filter 206 has a significantly larger filter area but does not require additional space beyond that anyway allocated to the expandable member 221 shown in FIG. 5B (deflated) and 5C (inflated). In this embodiment the air velocity over the filter element 206 may be as low as 10% relative to commonly used masks. A reduction of this order of magnitude is achieved owing to both the expandable member 221 and the peripheral surface area of the filter element 206 and assures significantly higher filtering efficiency.

The mask 204 may be formed of a material that is impermeable to air or may be of a typical structure adapted for air filter. In this configuration the one-way valve 228 blocks the air supply from the flexible pipe 231 and the one-way valve 227 allows direct access to the filtered air inside the expandable member 221. During the inhalation stage, breathing holes 234 allow a space 236 between the exterior of the expandable member 221 and the interior of chamber 202 to be freely filled by ambient air, thus maintaining the air inside the expandable member 221 at ambient pressure and avoiding a pressure drop inside the mask 204 during inhalation.

A one-way valve 227, such as a membrane valve, allows the flow of inhaled air via a flexible pipe 225 into a user's lungs. A one-way valve 228, such as a membrane valve, allows the flow of exhaled air via a flexible pipe 231 back to the filter 206. The inhalation and the exhalation portions of the filter 206 are separated by a separation wall 235.

The one-way valve 227 prevents the exhaled air flow from entering into the chamber 202 via pipe 225 and forces this air to flow via the pipe 231 to the exhaled air filter 206 where it is filtered. Simultaneous to the exhalation process, an air blower 208 conveys ambient air 207 through an outer surface of the filter 206 into an expandable member 221, which serves as an air capacitor that continually accumulates sufficient filtered air for the next inhalation. Breathing holes 234 maintain the air pressure in a space 236 between the exterior of expandable member 221 and the interior of the chamber 202 equal to the ambient air pressure. During the exhalation stage, the breathing holes 234 allow the air in the space between the exterior of expandable member 221 and the interior of chamber 202 to be freely discharged in order to maintain the air inside the expandable member 221 at the ambient pressure and to reduce the airflow resistance over the air blower 208 to a minimum.

FIG. 5B illustrates the filter element 206 split into two sections: an inhalation section on the right and an exhalation section on the left. In this embodiment, water vapor content of exhaled air is filtered far away from the mask 204 and therefor causes the wearer less discomfort.

The sequence of the breathing cycle introduced by the current embodiment is similar to that described above with reference to FIGS. 3A to 3D.

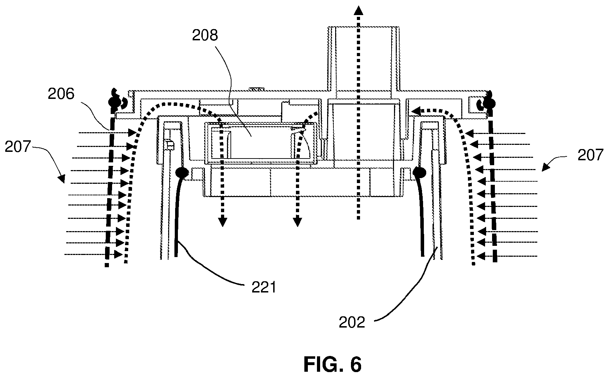

FIG. 6 is a detailed cross-section portion view of embodiment of FIG. 5. As it may be seen from this drawing, blower 208 collecting the filtered air from the space in between the filter 206 and chamber 202 and directs this air into the expandable member 221.

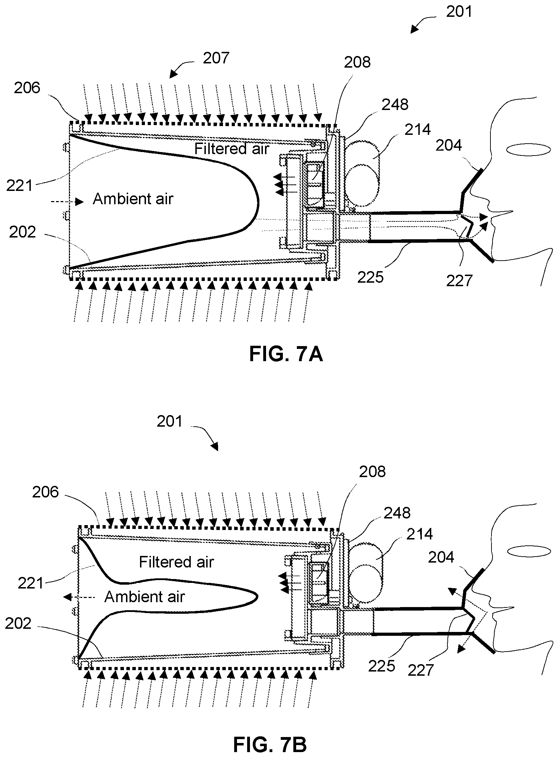

FIG. 7A illustrates a basic embodiment of a one-way module 201, having an expandable member 221 sealed to the base of a chamber 202, in an inhalation stage. A battery 214 is mounted on the exterior of the chamber 202 and connected to a controller 212, which controls the air blower 208, monitors battery voltage and current to the air blower 208 and collects environmental parameters, such as temperature and humidity.

An RFID reader may be interfaced to the controller 212 to identify the serial numbers of the filter 206 and blower 208 and to send this information via an embedded short-range communication (e.g. blue tooth, WiFi) to a service provider database.

In this embodiment the expandable member 221 is attached to the chamber 202 proximate the outer surface of the filter 206. This proximity may allow interconnection between the filter 206 and the expandable member 221, and moreover may allow the functionality of filter 206 and the expandable member 221 to be realized by a single structure, optionally made of the same material of the filter 206. As shown in the FIG. 7A, the exterior of the expandable member 221 is exposed to ambient air, and therefore inhalation via the short flexible pipe 225 should induce negligible pressure drop. By eliminating the pressure drop, the risk of contaminated air leakage of ambient air via the sealing lips of the mask 204 is negligible.

FIG. 7B illustrates the one-way module 201 of FIG. 7A, during exhalation. The filtered air is conveyed by the air blower 208 into the filtered air space captured between the expandable member 221 and the interior of the chamber 202. This space expands during the exhalation stage and reaches its maximum value prior to beginning of the inhalation stage.

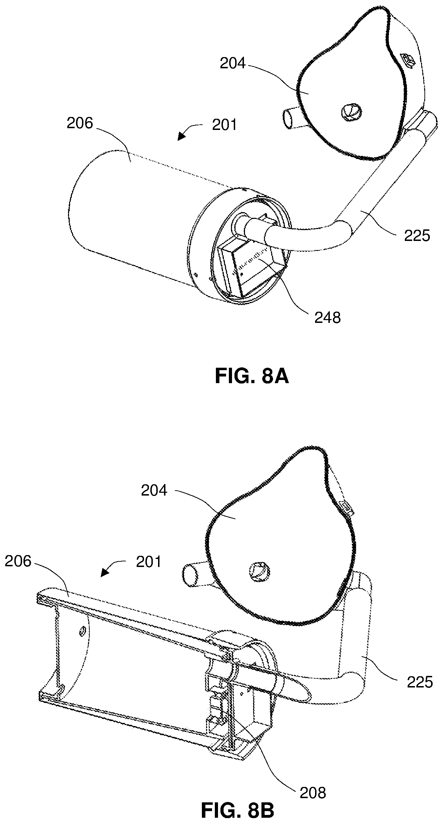

FIG. 8A illustrates pictorially an embodiment of the one-way module 201 of FIG. 7, coupled to a two-way mask 204 via a flexible pipe 225.

FIG. 8B shows a cross-section of the one-way module of FIG. 8A with a sampling module 238 (shown in FIG. 12, 13A and 13B) located inline the blower 208 airflow. The sampling module 238 can be attached to a mount 240 whenever the user or the service provider would like to monitor the system, module or the user's health condition. The sampling module 238 is configured to collect particles before or after filtering. When the sampling module 238 is located between the flexible pipe 231 and the exhalation filter 206 (FIG. 5), it will collect evidence of the user's health. When the sampling module 238 is located between the flexible pipe 225 and the inhalation filter 206, it will collect evidence of the performance of the one-way module 201. By attaching the sampling module 238 to the exterior of the filter 206, it will collect evidence of ambient air contamination or infection.

The sampling module 238 is configured to collect particles on a sampling surface that may be a conventional petri dish (FIG. 13A), or by a filter membrane, such as nylon membrane (not shown), glass surface or any other collecting surface or material capable of capturing and retaining the particles. The sampling principles of the above-mentioned modules 238 are different: a petri dish may capture the particles by its gel agar, while a filter membrane may capture the particles by its structure, glass or other surfaces that allow viruses to survive for a relatively long period, and may capture the particles by moisture condensation taking place in the one-way module during exhalation. The aggregated amount of air washing over the sampling module 238 over time, during its operation is significant (typically between 100 to 200 cc per second - at least 2.8 square meter per 8 hours) and therefore the probability of collecting contaminated particles, assuming that such particles are contained in the air, is very high. This allows a comprehensive monitoring process to be established on the following parameters: a) level of contamination of the ambient air; b) level of protection provided by the system 200 and its modules 201; c) level of contamination of the exhaled air.

Assuming broad implementation of the system 200 by organizations, such as hospitals, an overall contamination map of the entire organization can be established, on departmental and individual levels. The sampling module 238 can be configured based on the specific or broad monitoring target. For example, for Covid-19 fast response monitoring the sampling module 238 can be configured as a filter membrane capable of collecting particles of 0.1 micron. The sampling module 238 at the exhaled air outlet of the one-way module can provide the medical staff with a health indicator in different departments of hospital and trigger a specific investigation whenever desired. The sampling module 238 at the inhaled air inlet of the one-way module can provide a department air quality indicator and trigger a specific investigation, whenever desired. For broad monitoring the sampling module 238 can be configured as a petri dish.

The analysis of the sampling module 238 is derived from its configuration: the petri dish can be analyzed based on already well-established techniques. Filter membrane particulate content can be transferred to a liquid or to air by a back-pressure pulse and then be analyzed using known techniques.

To identify the sampling module 238 and to associate the sample with a specific user ID, a RFID chip (not shown) may be attached to the sample module 238. The pairing between the sampling module 238 and the one-way module 201 may be achieved by an RFID reader onboard the controller 212, or by an external RFID reader that collects the ID of the sampling module 238 and the ID of the one-way module 201 (also by RFID chip), and links both of them to a user and time stamp in the service provider database.

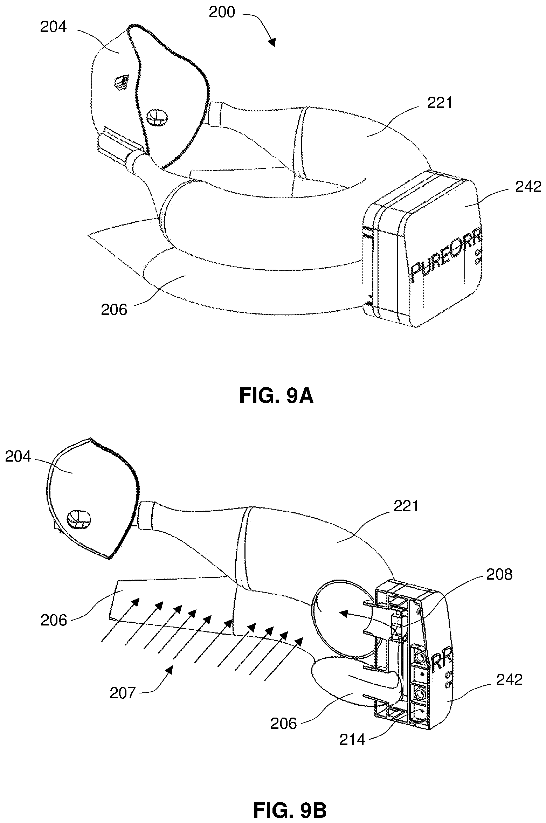

FIGS. 9A and 9B illustrate a basic embodiment of a two-way system 200 with expandable members in the inhalation and exhalation one-way modules. In this embodiment the expandable members 221 are separated from the filter elements 206. The first air blower 208 (FIG. 9B) inside the box 242 conveys inhaled air from the filter 206 to an expandable member 221 (on the right) and the second air blower (not shown) conveys exhaled air from the filter 206 to expandable member 221 (on the left).

It will be appreciated that the location of the inhalation and the exhalation one-way modules, on right or left in FIGS. 9A and 9B, as well as in the following figures, is arbitrary.

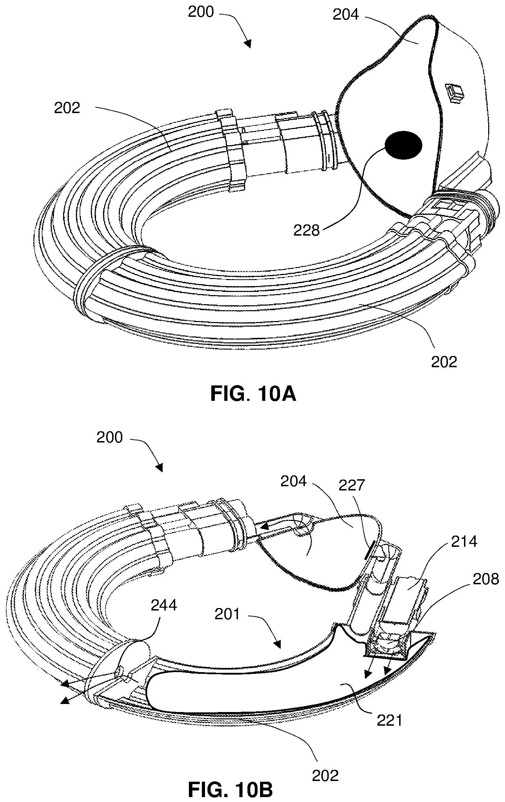

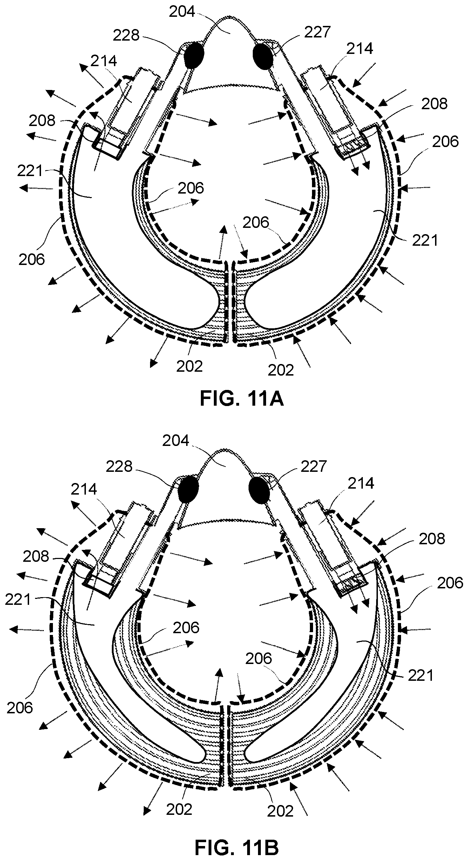

FIG. 10A illustrates a basic embodiment of a two-way system 200 having separate one-way modules 201 for inhalation and exhalation, respectively, each equipped with expandable members 221 embedded inside respective chambers 202. The principles of operation of each of the one-way modules are similar to those of the one-way modules shown in FIGS. 7 to 9. In this embodiment the chambers 202, the embedded expandable members 221 and the air blowers 208 are assembled and inserted inside the envelope of the filter 206 (FIGS. 11A and 11B). The envelope of the filter 206 can be formed from fabric and has a general shape of a neck pillow (flight pillow). To avoid airflow blockage by adhering of the filter fabric to the surface of the chambers 202, the chambers 202 are formed with a corrugated outer contour, thus allowing the air blower 208 to convey air between the filter element 206 and an exterior of the chamber 202, via the longitude tunnels created by the corrugations. The general profile of the chamber 202 may be elliptical, with a vertical axis shorter than the horizontal axis. The profile may vary in size from a typical shirt collar dimensions, to a neck pillow dimensions, in the extreme. The overall surface area of the filter element 206 of collar/pillow shape is at least twice that of any other face mask filter. The increased filter surface area allows for use of a lower power of air blower and provides improved filter performance.

FIG. 10B illustrates the system 200 of FIG. 10A with the one-way inhalation module 201 shown in cross-section.

Another important aspect of this embodiment is the design - the public may accept the shape, color and texture of the external fabric of the collar-like system 200 as a chic fashion item - that may encourage the public to protect itself without paying the penalty of poor appearance. To allow the user to change the appearance of the system 200, the system 200 can be inserted into fashion-collars or even into an entire shirt of different color, shape and texture. The fabric of fashion-collars should allow free airflow and low pressure drop. This requirement is easy to achieve because low-density fabrics are abundant at low production cost.

FIG. 11A illustrates a full cross-section of the system of FIG. 10A at the end of the exhalation stage and FIG. 11B illustrates a full cross-section of the same system at the end of the inhalation stage. The principles of operation of each of the one-way modules 201 of this embodiment are similar to those of the one-way modules 201 shown in the FIGS. 7 to 9. Thus, if we consider the left-hand one-way module 201 as the exhalation module, the action of this module is as follows: a) exhaled air inflates the left-hand expandable member 221. During this action, the air in the space outside the expandable member 221 and inside the chamber 202 is freely discharged into the atmosphere via an opening 244 (FIG. 10B), thus preventing pressure build-up inside the mask 204; b) the left-hand blower 208 continually deflates the left-hand expandable member 221 and conveys the exhaled air via the left-hand filter element 206 out of the system. The specific air flow (the ratio of the volumetric air flow to the area of the filter surface) is extremely low and therefore the power requirement of the blower 208 will be significantly lower and the filter 206 performance will be significantly higher.

Likewise, considering the right one-way module 201 as the inhalation module, its action is as follows: a) inhaled air deflates the right-hand expandable member 221. During this action the air in the space outside the right-hand expandable member 221 and inside the right-hand chamber 202 freely enters from the atmosphere via the opening 244 (FIG. 10B), thus preventing a pressure drop inside abovementioned space and inside the mask 204; b) the right-hand blower 208 continually inflates the right-hand expandable member 221 and conveys the air via the right-hand filter element 206 into the right-hand expandable member 221. As noted previously, the specific air flow is extremely low thus resulting in improved filter performance at lower power.

It should be noted that the inhalation and exhalation modules may be of similar size, as shown in the FIGS. 9 to 11, or may have different dimensions: a) unequal filter 206 area; b) expandable members 221 of unequal volume. In other words, the respective volumes of the two halves can be dedicated to expandable members and filters of different relative proportions. So, in one side, more of the available volume or of the available filter area can be allocated to the expandable member or to the filter area of one module at the expense of the other module and *vice versa.*

Possible examples for unequal allocations of the collar shaped system 200 of the embodiment of FIGS. 9 to 11 are: a) the whole volume of the expandable members 221 being allocated for the inhalation module only; b) the complete area of the filter 206 being allocated to inhalation module only; c) other allocations based on functional criteria (system for positively identified users) are valid as well.

FIG. 12 illustrates in cross-section and FIGS. 13A and 13B are detailed views of a power and monitoring module 248 integrated into the chamber 202. The power and monitoring module 248 shown in this embodiment allows efficient air sampling based on the proximity of the sampling module mount 240 to the air stream of the blower 208, thereby allowing measurement of inhalation and exhalation airflow and exhalation air temperature. The continuous operation of the blower 208 allows efficient particle collection over the sampling module 238. The power and monitoring module 248 is detachable, and therefore enables installation and removal of the sampling module 238 in a simple and a convenient way.

Airflow measurement can be performed using a miniature flow-meter 250 integrated inside the interface pipe 252 (FIG. 12) of the one-way module 201. To keep the interface pipe 252 sealed, the flow-meter blades' rotation velocity can be measured by an interaction between small magnets integrated in the tips of the flow-meter blades and a magnetic sensor interfaced to the controller 212 (FIGS. 13A and 13B). Another possibility to measure the flow-meter blades' rotation velocity is by a light emitting diode 254 (FIG. 13B) interacting with a reflective surface covering the blades (not shown). In this case the interface pipe 252 in the area of the diode 254 should be transparent to light.

To allow high sensitivity to extremely low airflow rates of inhalation and exhalation, the flow-meter blades may be rotatable about a thin string 256, in the range of 0.05 to 0.5 mm diameter, stretched between two mounts 258, along the pipe 252 axis, as illustrated in FIG. 13A.

The temperature of the exhaled air can be measured by a temperature sensor interfaced to the controller 212.

To sterilize the two-way system 200 a portable autoclave may be used (not shown). The autoclave may sterilize the system 200 by an external heat source (e.g. home oven), or by an embedded heat element configured to raise and maintain the autoclave temperature to a desired level. When an external heat source is used, the autoclave walls should be built from highly thermally conductive materials (e.g. aluminum). When an internal heat source is used, the autoclave walls should be thermally isolated. Another internal, broadly available, heat source is hot water that may be boiled and supplied from various water boiling devices.

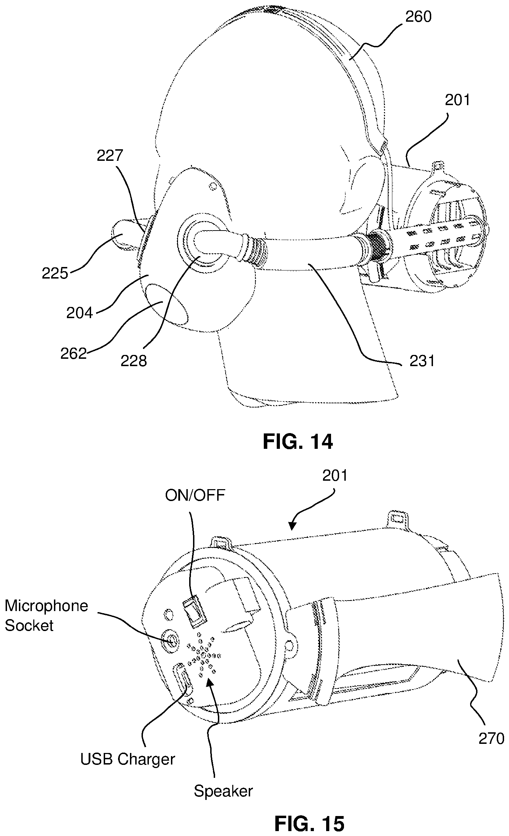

FIG. 14 illustrates use of the embodiment of the system of FIGS. 5A to 5C and 8A and 8B. The air mask 204 is adapted to be sealed to the user's face by flexible sealing lips stretched on the perimeter of the mask frame. One-way valves 227 and 228 have similar functionality to those shown in FIG. 5A to 5C. Flexible pipes 225 and 231 create a limited coupling force between the mask 204 and the face. The headband 260 stably supports the weight of the one-directional module 201 and system 200 over the user's head.

In order to adapt the air mask 204 to conduct the user's voice, a communication membrane 262 may be fitted at the front end of the mask. Alternatively, the air mask 204 may have a microphone, interfaced to an external speaker by wires or wirelessly.

FIG. 15 illustrates pictorially a detail of the one-way module 201 having a contoured neck rest 270, which sits comfortably against the back of the user's neck while being fluidly coupled to the mask at opposite ends via the expandable pipe 225 and the flexible pipe 231 as best seen in FIGS. 18A and 18B. On one end surface of the module 201 is an electronic assembly comprising an ON/OFF switch, microphone socket, speaker and USB port for connection of a battery charger.

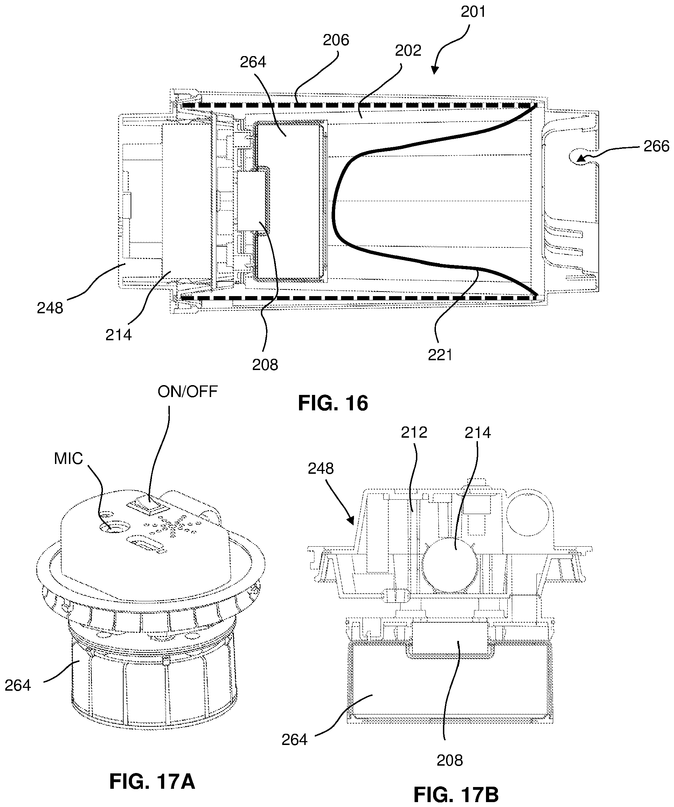

FIG. 16 shows a cross-section view of the one-way module 201 of FIG. 15. The module 201 has same structure and functionality as the module 201 of FIG. 7. A toxic gas absorption charcoal filter 264 may be coupled inline with the blower 208. The contact time between the air coming from the filter element 206, and the absorption filter 264 will be significantly longer owing to the low air velocity induced by the blower 208. Again, as explained regarding the UV disinfection and air filter, the reduced air velocity allowed by the expandable member 221 facilitates significantly higher disinfection, filtering and absorption efficiencies. As was explained with regard to FIG. 7, in this embodiment the proximity of the expandable member 221 to the filter 206 (on the right side of the drawing) allows interconnection between the filter 206 and the expandable member 221 to form a single structure, which may ease the installation requirements. A typical single structure consists of a cylindrical envelop filter 206 attached by either front or rear lips to lips of the expandable member 221,which may be in general a thin plastic bag of the type typically used for food storage.

Typically, the module 201, flexible pipe 225 and mask 204 are mutually interconnected prior to being worn. In order to create a single structure as shown in FIGS. 14 and 18A and 18B, a telescopic link 268 is connected to the flexible pipe 231 and is snap-fitted to a coupling element 266 (shown in FIG. 16) on one side of the module 201. The telescopic links 268, on the right and the left side of the system (FIGS. 18A and 18B), allow the system 200 to be adjusted to fit the user's head.

FIGS. 17A and 17B show a perspective and cross-section views of power and monitoring module 248 according to another embodiment. The module 248 incorporates the controller 212, the battery 214, the ON/OFF switch, the USB charger socket, the speaker and the microphone plug. The absorption filter 264 is optional and may be coupled by the user to the module 248 prior to being coupled to the module 201.

FIGS. 18A and 18B show the entire assembly of the system 200 over the user's head. To provide a soft and comfortable contact between the module 201 and the user's neck, a flexible support 270 can be coupled to the front side of the module 201. The support 270 and mask 204 mutually cooperate with the headband 260 to stabilize the system 200 on the user's head.

FIG. 19 is a rear view of the mask 204.

Real time monitoring of the system illustrated in any of FIGS. 1 to 15 may be performed as follows: a) actuating the air blowers 208; b) measuring inhalation and exhalation airflows; c) measuring the exhalation and inhalation temperatures; d) sampling the blower current; e) uploading the collected data by Bluetooth^{™} communication to a user's smartphone; f) uploading the data from the smartphone to a cloud application; e) analyzing the breathing profile of the user and defining the normal breathing flow-temperature profile; and g) looking for deviations and sending the user a message, if such deviation were detected.

Explanation: a) airflow increase may indicate an increase in the demand for higher amount of flow; b) airflow decrease may indicate a blocked filter 206 or degradation of lung functionality; c) exhalation air temperature increase indicates higher temperature of the user's body; d) frequent breathing, derived from the airflow measurement, may indicate a general condition of the user and together with the exhalation air temperature more significant indication of user health deviation.

Advanced monitoring can be achieved by transmitting the module 248 measurements, via the communication link, to a heart rate monitoring device (not shown), such as smart-watch, chest strip, or other, and synchronizing, in real-time, the transmitted data with the heart rate measurements. The combined measurements may then be analyzed and displayed to the user (e.g. on the smartphone). The analysis of the combined data can provide the user with heart-lung functionality indications in different situations, including sport activities, emergency activities and health monitoring activities. The added value of the system with the advance monitoring is dual: protecting the user and providing the user with health indicators. This is extremely important, especially during the pandemic periods, when fast detection of health conditions is required.

An alternative way is to adapt the system controller 212 to receive, via the communication link, the heart rate measurements from a heart rate monitoring device and to synchronize the measurements onboard the controller 212. In this case, the measurements can be analyzed and displayed over the user's smartphone and can be uploaded into the service provider's cloud database.

Note: in all embodiments, the service provider can be an employer organization (e.g. hospital) or a company selling the systems or a monitoring center that receives measured data from remote sources.

Off-line monitoring of the air quality may be performed as follows: a) actuating the air blowers 208; b) collecting infection particles by the sampling module 238 washed over by the blower air stream; c) associating the ID of the sampling module 238 with the user ID; d) removing the sampling module 238 and analyzing the collected content; e) associating the analysis with the user ID.

The description of the above embodiments is not intended to be limiting, the scope of protection being provided only by the appended claims.

In particular it should be noted that features that are described with reference to one or more embodiments are described by way of example rather than by way of limitation to those embodiments. Thus, unless stated otherwise or unless particular combinations are clearly inadmissible, optional features that are described with reference to only some embodiments are assumed to be likewise applicable to all other embodiments also.

It should also be noted that the claims constitute an integral part of the description and are intended to provide support for features that are recited in the claims but are not described in detail in the foregoing description.

It will be appreciated that when the filter is wrapped around the inside or exterior of the chamber to cover substantially the complete surface of the chamber, the surface area of the filter will be a function of the diameter of the chamber and its length. Without limitation, the length of the chamber is typically in the order of 15 cm and its diameter is typically in the order of 8 cm, such that the surface area of the sidewall of the chamber is πx15x8=377cm². This is approximately four times the area of a conventional air mask. It will further be appreciated that the filter can be of the form of a sleeve whose opposing edges formed a closed structure like a donut; but it can also be an open C-shaped structure or it may be spirally wound to form an overlapping structure that functions as a multi-layer structure.

## Claims

1. A device for enhancing efficiency of an air mask (204) wearable by a user to cover the mouth and nose of the user, the device comprising:
an elongated chamber (202) fluidly couplable at a proximal end thereof to an air opening (205, 205') of the mask (204),
a filter element (206) formed of flexible material that is wrapped around a sidewall of the chamber either internally or externally for filtering air flowing through the chamber in either direction,
a one-way valve (227, 228) mounted in association with the opening,
an air blower (208) configured to continuously supply air for inhalation or to discharge exhaled air,
**characterized in that**:
an expandable member (221) is fluidly coupled to the chamber:
the one-way valve (227, 228) is configured for allowing treated air inhaled by the user to pass from the chamber to the mask or for allowing air exhaled by the user to pass from the mask to the chamber,
the expandable member (221) is operably coupled to the one-way valve for directing air through the filter during inhalation or exhalation;
whereby if the opening is configured to be an air inlet, then during inhalation air is constrained to flow into the chamber and inflate the expandable member (221) with treated air ready for inhalation prior to flowing out through the opening into the user's lungs; and
if the opening is an air outlet, then during exhalation air is constrained to flow from the user's lungs into the chamber and inflate the expandable member (221) with exhaled air prior to being discharged as treated air into the environment.

2. The device according to claim 1, wherein the filter element is made of a thin filter media folded or rolled to create a hollow structure having openings at opposite ends, and wherein optionally one of said openings is fluidly coupled to an opening of the expandable member (221) in a unitary construction.

3. The device according to any one of claims 1 to 3, wherein a profile of the chamber is designed and dimensioned for insertion into a shirt collar of an entire shirt or wherein the chamber is designed as a canister interconnected with the mask by two flexible pipes.

4. The device according to any one of the preceding claims, including at least one UV source selected from a group consisting of: bulbs or LEDs and any combination thereof for either (i) purifying air prior to inhalation if the one-way valve is configured for allowing purified air inhaled by the user to pass from the chamber to the mask or (ii) purifying exhaled air prior to its being released to the environment if the one-way valve is configured for allowing air exhaled by the user to pass from the mask to the chamber.

5. The device according to any one of the preceding claims, wherein the filter element is configured to be worn on the user's head.

6. The device according to any one of the preceding claims, wherein the air blower is configured to move the air through the chamber and inflate or deflate the expandable member with a volume of air needed for at least one inhalation or exhalation, or wherein the air blower is configured to provide a minimum airflow sufficient to meet breathing demand, thus treating incoming air to the chamber at the lowest possible flowrate to maximize the treatment efficiency.

7. The device according to any one of the preceding claims, wherein the expandable member is cyclically inflated or deflated for each successive breathing cycle.

8. The device according to any one of the preceding claims, wherein the expandable member is configured to be either inflated by exhalation and deflated by the air blower, or inflated by the air blower and deflated by inhalation.

9. The device according to any one of the preceding claims, wherein the expandable member is disposed inside the chamber, or is coupled to an exterior of the chamber.

10. The device according to any one of the preceding claims, wherein the chamber forms an integral unit with an air mask.

11. A system comprising an air mask coupled to the device according to any one of the preceding claims and further including at least one sampling module coupled to at least one of an exterior or an interior of the device and each being configured to collect air particles for collecting evidence of ambient air contamination or infection prior to inhalation or for collecting evidence of the user's health after exhalation, respectively.

12. The system according to 11, wherein the at least one sampling module and filter form an integral unit.

13. The system according to claim 11 or 12, wherein:
the air blower (208) is configured to continuously supply air for inhalation, while creating an overpressure inside the air mask, and
there is further provided a controller (212) configured to sense motor current of the air blower and to convey the current data to a remote computer or to a central database so as to allow a measured current deviation from a threshold value to be interpreted as a blocked filter, breathing cycle stagnation points or motor malfunction.

14. The system according to claim 13, wherein the controller includes a microprocessor and respective sensors capable of sensing air temperature, air humidity, atmospheric pressure, battery voltage and health status, blower ID, sampling module ID, inhalation flowrate, exhalation flowrate and exhalation air temperature, and optionally wherein the controller has a communication channel capable of transmitting, at least indirectly, a unique ID and information gathered by the sensors to a central data base or a smartphone.

15. The system according to any one of claims 11 to 14, wherein the air mask is configured for sealing to the user's face by a flexible membrane stretched on a perimeter of a frame of the mask, and a central part of the membrane has a cutout adapted to fit the nose and the lips size and shape of the user, optionally wherein the frame of the mask is configured for attachment to membranes of different size and having cutouts of different size and shape, thus allowing the user to choose one that will fit his or her face best, and optionally wherein the air mask has a communication membrane adapted to conduct the user's voice or has a microphone for wired or wireless interfacing to an external speaker.

## Patentansprüche

1. Ein Gerät zur Verbesserung der Effizienz einer Atemschutzmaske (204), die von einem Benutzer getragen wird, um Mund sowie Nase zu bedecken, wobei das Gerät umfasst:
eine längliche Kammer (202), die an ihrem proximalen Ende fluidisch mit einer Luftöffnung (205, 205') der Maske (204) verbindbar ist,
ein Filterelement (206) aus flexiblem Material, das entweder intern oder extern um die Seitenwand der Kammer gewickelt ist, um Luft, die in beide Richtungen durch die Kammer strömt, zu filtern,
ein Einwegventil (227, 228), das in Verbindung mit der Öffnung montiert ist, ein Luftgebläse (208), das so konfiguriert ist, dass es kontinuierlich Luft zur Einatmung bereitstellt oder ausgeatmete Luft abführt,
**gekennzeichnet dadurch, dass**:
ein dehnbares Element (221) fluidisch mit der Kammer verbunden ist:
das Einwegventil (227, 228) so konfiguriert ist, dass es behandelte Luft, die vom Benutzer eingeatmet wird, aus der Kammer in die Maske leitet oder ausgeatmete Luft aus der Maske in die Kammer leitet,
das dehnbare Element (221) funktionsfähig mit dem Einwegventil verbunden ist, um Luft während der Einatmung oder Ausatmung durch den Filter zu leiten;
wobei, wenn die Öffnung als Lufteinlass konfiguriert ist, die Luft während der Einatmung in die Kammer gezwungen ist zu strömen und das dehnbare Element (221) mit behandelter Luft aufzublasen, die zur Einatmung bereitsteht, bevor sie durch die Öffnung in die Lunge des Benutzers strömt; und
wenn die Öffnung ein Luftauslass ist, die Luft während der Ausatmung aus der Lunge des Benutzers in die Kammer gezwungen ist zu strömen und das dehnbare Element (221) mit ausgeatmeter Luft aufzublasen, bevor sie als behandelte Luft in die Umgebung abgegeben wird.

2. Das Gerät nach Anspruch 1, wobei das Filterelement aus einem dünnen Filtermaterial besteht, das gefaltet oder gerollt ist, um eine hohle Struktur mit Öffnungen an gegenüberliegenden Enden zu bilden, und wobei optional eine dieser Öffnungen fluidisch mit einer Öffnung des dehnbaren Elements (221) in einer einheitlichen Konstruktion verbunden ist.

3. Das Gerät nach einem der Ansprüche 1 bis 3, wobei das Profil der Kammer so gestaltet und dimensioniert ist, dass sie in den Kragen eines Hemds oder in ein vollständiges Hemd eingefügt werden kann, oder wobei die Kammer als Kanister ausgeführt ist, der mit der Maske durch zwei flexible Schläuche verbunden ist.

4. Das Gerät nach einem der vorangehenden Ansprüche, umfassend mindestens eine UV-Quelle, die aus einer Gruppe, bestehend aus Lampen oder LEDs und beliebigen Kombinationen davon, ausgewählt ist, um entweder (i) Luft vor der Einatmung zu reinigen, wenn das Einwegventil so konfiguriert ist, dass gereinigte Luft vom Benutzer aus der Kammer in die Maske geleitet wird, oder (ii) ausgeatmete Luft vor ihrer Freisetzung in die Umgebung zu reinigen, wenn das Einwegventil so konfiguriert ist, dass ausgeatmete Luft aus der Maske in die Kammer erlaubt wird zu strömen.

5. Das Gerät nach einem der vorangehenden Ansprüche, wobei das Filterelement so gestaltet ist, dass es am Kopf des Benutzers getragen werden kann.

6. Das Gerät nach einem der vorangehenden Ansprüche, wobei das Luftgebläse so konfiguriert ist, dass es die Luft durch die Kammer bewegt und das dehnbare Element mit einem Luftvolumen aufbläst oder entleert, das für mindestens eine Einatmung oder Ausatmung erforderlich ist, oder wobei das Luftgebläse so konfiguriert ist, dass es einen minimalen Luftstrom bereitstellt, der ausreicht, um den Atembedarf zu decken, wodurch die einströmende Luft in die Kammer mit der niedrigstmöglichen Durchflussrate behandelt wird, um die Behandlungseffizienz zu maximieren.

7. Das Gerät nach einem der vorangehenden Ansprüche, wobei das dehnbare Element zyklisch für jeden aufeinanderfolgenden Atemzyklus aufgeblasen oder entleert wird.

8. Das Gerät nach einem der vorangehenden Ansprüche, wobei das dehnbare Element konfiguriert ist, entweder durch die Ausatmung aufgeblasen zu werden und durch das Luftgebläse entleert zu werden oder durch das Luftgebläse aufgeblasen und durch die Einatmung entleert zu werden.

9. Das Gerät nach einem der vorangehenden Ansprüche, wobei das dehnbare Element innerhalb der Kammer angeordnet ist oder an einem äußeren Bereich der Kammer angebracht ist.

10. Das Gerät nach einem der vorangehenden Ansprüche, wobei die Kammer eine integrale Einheit mit einer Atemschutzmaske bildet.

11. Ein System, umfassend eine Atemschutzmaske, die mit dem Gerät nach einem der vorangehenden Ansprüche verbunden ist, und umfassend ferner mindestens ein Probenahmemodul, das an mindestens einem von der Außen- oder Innenseite des Geräts gekoppelt ist und jeweils so konfiguriert ist, dass es Luftpartikel sammelt, um Beweise für Verunreinigungen oder Infektionen der Umgebungsluft vor der Einatmung zu sammeln oder um Beweise für die Gesundheit des Benutzers nach der Ausatmung zu sammeln.

12. Das System nach Anspruch 11, wobei das mindestens eine Probenahmemodul und der Filter eine integrale Einheit bilden.

13. Das System nach Anspruch 11 oder 12, wobei:
das Luftgebläse (208) so konfiguriert ist, dass es kontinuierlich Luft zur Einatmung liefert und gleichzeitig einen Überdruck innerhalb der Atemschutzmaske erzeugt, und
weiterhin ein Controller (212) bereitgestellt ist, der konfiguriert ist, den Motorstrom des Luftgebläses zu messen und die Stromdaten an einen entfernten Computer oder eine zentrale Datenbank weiterzuleiten, um eine gemessene Stromabweichung von einem Schwellenwert als blockierten Filter, Atemzyklus-Stagnationspunkte oder Motorfehlfunktion zu interpretieren.

14. Das System nach Anspruch 13, wobei der Controller einen Mikroprozessor und entsprechende Sensoren umfasst, die in der Lage sind, Lufttemperatur, Luftfeuchtigkeit, Atmosphärendruck, Batteriespannung, Gesundheitsstatus, Gebläse-ID, Probenahmemodul-ID, Einatmungsflussrate, Ausatmungsflussrate und Ausatmungslufttemperatur zu erfassen, und optional, wobei der Controller über einen Kommunikationskanal verfügt, der in der Lage ist, mindestens indirekt eine eindeutige **ID** und die von den Sensoren erfassten Informationen an eine zentrale Datenbank oder ein Smartphone zu übertragen.

15. Das System nach einem der Ansprüche 11 bis 14, wobei die Atemschutzmaske so konfiguriert ist, dass sie mithilfe einer flexiblen Membran, die entlang des Umfangs eines Rahmens der Maske gespannt ist, am Gesicht des Benutzers abdichtet, und ein zentraler Teil der Membran einen Ausschnitt aufweist, der an die Größe und Form der Nase und Lippen des Benutzers angepasst ist; wobei optional der Rahmen der Maske für den Einsatz mit Membranen unterschiedlicher Größe und mit Ausschnitten unterschiedlicher Größe und Form konfiguriert ist, sodass der Benutzer eine auswählen kann, die am besten zu seinem Gesicht passt, wobei optionanl die Atemschutzmaske eine Kommunikationsmembran aufweist, die die Stimme des Benutzers leitet, oder ein Mikrofon aufweist, das für eine kabelgebundene oder kabellose Verbindung zu einem externen Lautsprecher geeignet ist.

## Revendications

1. Dispositif pour améliorer l'efficacité d'un masque à air (204) pouvant être porté par un utilisateur pour couvrir la bouche et le nez de l'utilisateur, le dispositif comprenant :
une chambre allongée (202) pouvant être couplée de manière fluidique au niveau d'une extrémité proximale de celle-ci à une ouverture d'air (205, 205') du masque (204),
un élément filtrant (206) formé d'un matériau flexible qui est enroulé autour d'une paroi latérale de la chambre, soit à l'intérieur, soit à l'extérieur, pour filtrer l'air qui traverse la chambre dans l'une ou l'autre direction,
une valve unidirectionnelle (227, 228) montée en association avec l'ouverture,
un ventilateur (208) configuré pour fournir en continu de l'air pour l'inhalation ou pour évacuer l'air expiré, **caractérisé en ce que** :
un membre extensible (221) est couplé fluidiquement à la chambre :
la valve unidirectionnelle (227, 228) est configurée pour permettre à l'air traité inhalé par l'utilisateur de passer de la chambre au masque ou pour permettre à l'air expiré par l'utilisateur de passer du masque à la chambre,
le membre extensible (221) est couplé de manière opérationnelle à la valve unidirectionnelle pour diriger l'air à travers le filtre pendant l'inhalation ou l'expiration ;
selon lequel si l'ouverture est configurée pour être une entrée d'air, alors pendant l'inhalation l'air est contraint de s'écouler dans la chambre et de gonfler le membre extensible (221) avec de l'air traité prêt à être inhalé avant de s'écouler par l'ouverture dans les poumons de l'utilisateur ; et
si l'ouverture est une sortie d'air, alors pendant l'expiration, l'air est contraint de s'écouler des poumons de l'utilisateur dans la chambre et de gonfler le membre extensible (221) avec de l'air expiré avant d'être évacué dans l'environnement sous forme d'air traité.

2. Dispositif selon la revendication 1, dans lequel l'élément filtrant est constitué d'un mince média filtrant plié ou roulé pour créer une structure creuse ayant des ouvertures aux extrémités opposées, et dans lequel, éventuellement, l'une desdites ouvertures est couplée fluidiquement à une ouverture du membre extensible (221) dans une construction unitaire.

3. Dispositif selon l'une quelconque es revendications 1 à 3, dans lequel un profil de la chambre est conçu et dimensionné pour être inséré dans le col de chemise d'une chemise entière ou dans lequel la chambre est conçue comme une boîte reliée au masque par deux tuyaux flexibles.

4. Dispositif selon l'une quelconque des revendications précédentes, comprenant au moins une source UV choisie dans un groupe composé de : ampoules ou LED et toute combinaison de celles-ci pour (i) purifier l'air avant l'inhalation si la valve unidirectionnelle est configurée pour permettre à l'air purifié inhalé par l'utilisateur de passer de la chambre au masque ou (ii) purifier l'air expiré avant sa libération dans l'environnement si la valve unidirectionnelle est configurée pour permettre à l'air expiré par l'utilisateur de passer du masque à la chambre.

5. Dispositif selon l'une quelconque des revendications précédentes, dans lequel l'élément filtrant est configuré pour être porté sur la tête de l'utilisateur.

6. Dispositif selon l'une quelconque des revendications précédentes, dans lequel le ventilateur est configuré pour déplacer l'air dans la chambre et gonfler ou dégonfler le membre extensible avec un volume d'air nécessaire pour au moins une inhalation ou une expiration, ou dans lequel le ventilateur est configuré pour fournir un débit d'air minimum suffisant pour répondre à la demande de respiration, traitant ainsi l'air entrant dans la chambre au débit le plus bas possible pour maximiser l'efficacité de traitement.

7. Dispositif selon l'une quelconque des revendications précédentes, dans lequel le membre extensible est gonflé ou dégonflé de manière cyclique pour chaque cycle respiratoire successif.

8. Dispositif selon l'une quelconque des revendications précédentes, dans lequel le membre extensible est configuré pour être soit gonflé par l'expiration et dégonflé par le ventilateur, soit gonflé par le ventilateur et dégonflé par l'inhalation.

9. Dispositif selon l'une quelconque des revendications précédentes, dans lequel le membre extensible est disposé à l'intérieur de la chambre ou est couplé à l'extérieur de la chambre.

10. Dispositif selon l'une quelconque des revendications précédentes, dans lequel la chambre fait partie intégrante d'un masque à air.

11. Système comprenant un masque à air couplé au dispositif selon l'une quelconque des revendications précédentes et comprenant en outre au moins un module d'échantillonnage couplé à au moins un de l'extérieur ou de l'intérieur du dispositif, et chacun étant configuré pour collecter des particules d'air afin de recueillir des preuves de la contamination ou de l'infection de l'air ambiant avant l'inhalation ou de recueillir des preuves de l'état de santé de l'utilisateur après l'exhalation, respectivement.

12. Système selon la revendication 11, dans lequel ledit au moins un module d'échantillonnage et un filtre forment une unité intégrale.

13. Système selon la revendication 11 ou 12, dans lequel :
le ventilateur (208) est configuré pour fournir continuellement de l'air pour l'inhalation, tout en créant une surpression à l'intérieur du masque à air, et
il est également prévu un contrôleur (212) configuré pour détecter un courant de moteur du ventilateur et pour transmettre les données de courant à un ordinateur distant ou à une base de données centrale de manière à permettre qu'un écart de courant mesuré par rapport à une valeur seuil soit interprété comme un filtre bloqué, des points de stagnation de cycle respiratoire ou un dysfonctionnement de moteur.

14. Système selon la revendication 13, dans lequel le contrôleur comprend un microprocesseur et des capteurs respectifs capables de détecter la température de l'air, l'humidité de l'air, la pression atmosphérique, la tension de la batterie et son état de santé, l'identifiant du ventilateur, l'identifiant du module d'échantillonnage, le débit d'inhalation, le débit d'exhalation et la température de l'air d'exhalation, et éventuellement dans lequel le contrôleur dispose d'un canal de communication capable de transmettre, au moins indirectement, un identifiant unique et les informations recueillies par les capteurs à une base de données centrale ou à un téléphone intelligent.

15. Système selon l'une quelconque des revendications 11 à 14, dans lequel le masque à air est configuré pour être scellé au visage de l'utilisateur par une membrane souple tendue sur un périmètre d'un cadre du masque, et une partie centrale de la membrane a une découpe adaptée à la taille et à la forme du nez et des lèvres de l'utilisateur, éventuellement, dans lequel le cadre du masque est configuré pour être fixé à des membranes de différentes tailles et ayant des découpes de différentes tailles et formes, permettant ainsi à l'utilisateur de choisir celle qui s'adaptera le mieux à son visage, et éventuellement, dans lequel le masque à air est doté d'une membrane de communication adaptée pour conduire la voix de l'utilisateur ou d'un microphone pour une interface câblée ou sans fil avec un haut-parleur externe.
